(19) Europäisches Patentamt
European Patent Office
Office européen des brevets

(11) Publication number: **0 054 294**
**B1**

(12) # EUROPEAN PATENT SPECIFICATION

(45) Date of publication of patent specification: **16.10.85**

(51) Int. Cl.⁴: **C 07 D 229/00,**
C 07 C 119/055

(21) Application number: **81110428.0**

(22) Date of filing: **14.12.81**

(54) **Process for converting isocyanato groups into carbodiimido and/or urethonimino groups.**

(30) Priority: **16.12.80 IT 2668680**

(43) Date of publication of application:
**23.06.82 Bulletin 82/25**

(45) Publication of the grant of the patent:
**16.10.85 Bulletin 85/42**

(84) Designated Contracting States:
**AT BE DE FR GB NL**

(56) References cited:
**GB-A-1 523 962**
**US-A-4 014 935**
**US-A-4 031 026**
**US-A-4 284 730**

**Encyclopedia of Chemical Technology, 3rd Edition, Vol. 2, pages 225 and 230-233**

(73) Proprietor: **Montedison S.p.A.**
**Patents & Licensing Dept. Foro Buonaparte, 31**
**P.O. Box 10528**
**I-20121 Milan (IT)**

(72) Inventor: **Felice, Riva Mario, Dr. Chem.**
**14d Via Lanfranco della Pila**
**Milano (IT)**

(74) Representative: **Schmied-Kowarzik, Volker, Dr. et al**
**Patentanwälte Dr. V. Schmied-Kowarzik Dipl.-Ing. G. Dannenberg Dr. P. Weinhold Dr. D. Gudel Dipl.-Ing. S. Schubert Dr. P. Barz**
**Siegfriedstrasse 8**
**D-8000 München 40 (DE)**

**EP 0 054 294 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to a process for partially or thoroughly converting isocyanato groups, contained in organic compounds such as polyisocyanates, into carbodiimido and/or urethonimino groups.

In particular, this invention relates to the preparation of liquid polyisocyanates stable to storing.

It is known that the organic polyisocyanates, in particular the diisocyanates such as diphenylmethane diisocyanate, as well as the polymeric derivatives thereof, as compounds necessary in the preparation of polyurethanes, are relatively little stable to storing. In fact, if kept in the liquid form, they lead to the formation of precipitates or even to solidification.

A method of preparing stable polyisocyanates is that of modifying them by partially transforming their isocyanato functional groups into carbodiimido functional groups, with the production of a liquid modified polyisocyanate suitable for storing under ambient conditions.

It is known that the organic isocyanates are capable of being converted in part or in whole into carbodiimides in consequence of prolonged heating at high temperatures, generally exceeding 180°C.

According to said carbodiimidation reaction, a carbodiimido group (—N=C=N—) and a molecule of carbon dioxide are obtained from two isocyanate equivalents.

With a view to reducing both temperature and reaction times, i.e. to increasing the reaction rate, it was suggested to employ a variety of catalysts, out of which we cite, by way of example: triethylphosphate, hexamethyl-phosphoramide, tris(chloromethyl)-phosphine-oxide, 3-methyl-1-phenyl-2-phospholene-1-oxide, n-butyltriphenylphosphonium bromide, tetraoctylzirconate, tris(isocyanate-hexyl)-biuret, tetraisopropyltitanate, triphenylarsine, ferropentacarbonyl, bis(β-chloroethyl)-vinyl phosphonate and organic compounds containing isocyanato groups and biuret, urethane, urea, amido, allophanate, isocyanurate, urethdione, urethonimine groups.

In practice the aforesaid catalysts may be considered as belonging to two classes: the ones which are active also at temperatures below room temperature, such as for example 3-methyl-1-phenyl-2-phospholene-1-oxide, and the ones which are active at high temperatures, generally above 150°C, with a minimum activity or without any activity at room temperature, such as for example triethylphosphate.

To stop the carbodiimidation reaction at the desired conversion degree it would be necessary, therefore, in the case of the catalysts belonging to the former class, to deactivate or remove them, while for the ones belonging to the latter class it could be sufficient in principle to drastically lower the reaction temperature.

However, the use of said catalysts exhibits several drawbacks which are mainly due to the fact that it is difficult to remove them from the finished modified isocyanato product. In fact, the presence of said catalysts in the finished modified isocyanato product, in relation also to the storing, handling, transport conditions and to the successive utilization conditions, may cause, chiefly in the case of the high-activity catalysts, a further progress of the carbodiimidation reaction. By consequence, variations in some characteristics of the product may occur, among which, for example, a variation in the NCO titer and in the viscosity, as well as an evolution of gaseous carbon dioxide which can cause dangerous pressure increases in the storage tanks.

To overcome these problems various attempts have been made.

US—A—4 014 935 discloses a process wherein, after the desired degree of conversion is reached, the catalyst is deactivated by addition of a compound which reacts with the catalyst, thereby rendering it inactive. Alternatively, in order to remove the catalyst from the reaction mixture said reference describes the addition of solid substrates, e.g. silicious earths, which absorb the catalyst and may thereafter be separated by filtration or other techniques.

The process described in GB—A 1 523 962 uses a catalyst which is already fixed to an insoluble high molecular weight matrix, such as an ion exchange resin, by ionic bonding.

Said heterophase catalysts, however, have the drawback that their preparation is difficult and expensive since it comprises, for example, the production of isocyanate insoluble polymers containing active groups of the previously listed catalysts, or the fixing of said active catalytic groups to an inert solid substrate.

It has now been found that it is possible to partly or thoroughly convert the isocyanato groups, contained in organic compounds, into carbodiimido and/or urethonimino groups, in particular to prepare polyisocyanates containing carbodiimido and/or urethonimino groups, without the drawbacks mentioned hereinbefore, by employing, as carbodiimidation reaction catalysts, solid insoluble inorganic substances having a high specific surface and a high porosity, and containing, as main components, silica and/or alumina.

Thus, object of the present invention is a process for the partial or total conversion of isocyanato groups, contained in organic compounds, into carbodiimido and/or urethonimino groups by heating compounds containing free isocyanato functional groups, such as the polyisocyanates, at high temperatures in the presence of catalysts, the process being characterized in that heating occurs at temperatures in the range of from 140° to 300°C in the presence of a catalyst composed of a solid insoluble inorganic substance, of acid nature, having a specific surface of at least 2 $m^2/g$, selected from silica, alumina and of mixtures or derivatives thereof in which these two oxides are united and/or mixed with each other and/or with alkali or alkaline-earth metal oxides.

The substances utilized as catalysts, according to the present invention, are generally endowed with a high specific surface, preferably higher than 10 $m^2/g$, and with a high porosity, they may be of natural or synthetic origin and may be used in the form of powders, granules, spherical or cylindrical particles and the like.

Among such substances, besides silica and alumina, also crystalline silico-aluminates having zeolitic structure, also known as molecular sieves, may be cited.

As is known, such substances are generally activated, prior to use, by heating them in anhydrous inert atmosphere or in the presence of a defined amount of water.

The activity for each type of such catalysts depends also on the mode according to which the activation process is conducted.

The catalysts of the present invention can be activated by heating at 100—600°C, preferably at 140—350°C, in a dry nitrogen or air stream; temperature can be kept constant or gradually increased starting from room temperature.

Before activation the catalysts may be optionally subjected to a pretreatment with moist air at room temperature, or with heated air or nitrogen, having a certain humidity content.

Said catalysts are easily available as commercial products, are moderately expensive and, being in the solid form, are easily removable from the isocyanate reaction product, for example by filtration, decantation, centrifugation. Furthermore they can be reused for subsequent carbodiimidation cycles.

The complete removal of the catalysts makes it possible to prevent any further forming of carbodiimide during the storage of the modified isocyanate products.

The organic compounds containing free isocyanato functional groups, employable in the process according to this invention, may be monofunctional such as tolylisocyanate, cyclohexylisocyanate, or polyfunctional, such as the polyisocyanates of the aromatic, aliphatic and cycloaliphatic types, among which there are to be cited, for example, 4,4'-diphenylmethane diisocyanate, 4,4'-diphenyl-dimethylmethane diisocyanate, 2,4-toluene diisocyanate (either alone or in admixture with its 2,6-isomer), 1,6-hexane-diisocyanate, 1,4-butane-diisocyanate, 4,4-dicyclo-hexyl-methane diisocyanate, isoforone diisocyanate, 2,4-methylcyclohexane diisocyanate, p-xylene diisocyanate, triphenyl-methane-4,4',4''-triisocyanate, polymeric polyisocyanates and the products, containing free NCO, obtained by partial condensation between a polyisocyanate and compounds containing activated hydrogen atoms.

In particular, by using, as an organic polyisocyanate, the diphenylmethane diisocyanate (MDI) or the polymeric diphenylmethane diisocyanate (crude-MDI) having a high content of diphenylmethane diisocyanate, it is possible to obtain liquid carbodiimide-modified polyisocyanates, which are stable to storing for long time at room temperature and do not lead to the formation of precipitates or to solidification, as conversely happens in the case of the not modified starting polyisocyanates.

The process conforming to the present invention is conducted by contacting, according to any technique, the organic polyisocyanate with the catalyst and by maintaining the whole at temperature in the range of from 140° to 300°C, preferably from 180° to 260°C.

The reaction can be started, or even thoroughly conducted in the presence of any organic solvent unreactive towards isocyanato groups, such as for example chlorobenzene, chloroform, diglyme, methylene chloride, N-N-dimethylacetamide.

The reaction progress degree, i.e. the forming of carbodiimide at the expense of the isocyanato functional groups, can be easily checked volumetrically by measuring the amount of evolved carbon dioxide.

As soon as the conversion of the organic polyisocyanate to carbodiimide reaches the value desired, the reaction can be stopped by removing the catalyst from the modified isocyanate product or by suitably lowering the temperature of the reacted mass. In the latter case, the catalyst can be successively removed according to any separation technique, such as, for example, filtration, decantation, centrifugation.

The so separated catalyst may be subsequently utilized again.

The reaction progress can be determined also by means of other techniques, for instance by checking the refractive index, the infrared spectrum or the NCO titer.

When operating discontinuously it is preferable to suspend the catalyst, by means of intense stirring, in the liquid isocyanate mass.

The process may be conducted also continuously, using the solid catalyst as a fixed bed in a column, to which polyisocyanate is continuously fed.

By properly choosing the type and caking degree of the catalyst, its particle size and filling degree in the column, as well as the temperature, pressure and flow rate of the polyisocyanate in the column, it is possible to achieve the desired conversion of the isocyanato functional groups into carbodiimide.

It is possible to use more columns arranged in series, and it is also possible to recycle the isocyanate product till attaining the desired conversion.

The continuous process with catalyst fixed bed is particularly advantageous because the final modified isocyanate product results already separated from the catalyst which remains in the reaction column.

According to this process it is possible to directly obtain a modified polyisocyanate at the desired conversion degree, namely with the carbodiimide content which is useful for its application, or to reach a higher conversion of the isocyanato functional groups into carbodiimide than the necessary one, and then to dilute the resulting product with the unmodified polyisocyanate or with another type of polyisocyanate,

up to the carbodiimide content to be obtained.

The advantage resulting therefrom consists in the fact that not only remarkably lower amounts of polyisocyanate are made to react, but also that the obtained product, having a high carbodiimide content, is capable of providing, by dilution, a wide range of modified polyisocyanates with a carbodiimide content varying according to the different requirements.

Dilution may be performed directly in the reaction vessel or column by using unreacted polyisocyanate maintained at low temperature, with the further advantage of a sudden cooling of the reacting mass which stops the reaction and reduces as much as possible the formation of undesired urethdionic by-products.

It is to be pointed out that the polyisocyanates containing carbodiimide can form an adduct (urethonimine), by interaction between an isocyanato group and a carbodiimido group according to the following scheme:

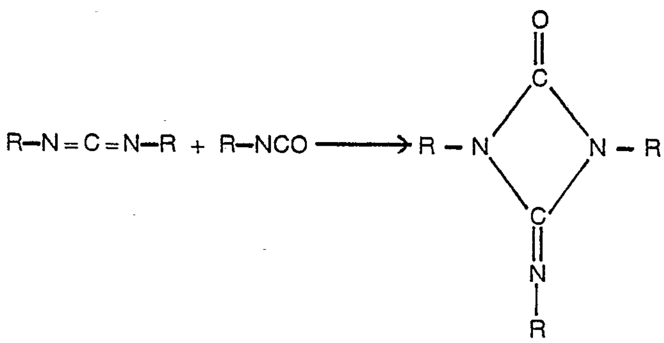

Such reaction tends to occur spontaneously at room temperature and is easily reversible; for example by heating, the adduct is decomposed while restoring the isocyanate and carbodiimide groups. Therefore such an adduct can be considered a "masked" isocyanate.

The modified polyisocyanates obtained according to the process of the present invention may therefore contain, generally after a more or less prolonged storing period, urethonimino groups.

The titer of modified polyisocyanates which takes the masked isocyanate into account is defined as total "NCO", while the one which does not take it into account is defined as "apparent NCO" and it will be of course always lower than the "total NCO".

The following examples are given to better illustrate this invention.

In such examples, the carbon dioxide evolved during the reaction was measured volumetrically or by titrating, with hydrochloric acid, the caustic soda aqueous solution on which it was collected.

The percentage of apparent NCO was determined according to method ASTM D—1638 or by infrared spectrophotometry.

The percentage of total NCO was determined according to method ASTM D—1638 which, however, was modified by heating at reflux the mass being titred, before adding isopropyl alcohol, in order to be able to titrate also the isocyanate masked as urethonimine.

The viscosity was measured at 25°C by using Brookfield torsion viscometer.

Besides the presence of little amounts of urethdione (isocyanate dimer), no significant presence of other by-products, such as urea derivatives and isocyanurates, was detected, what proves that the catalyst employed according to the invention is highly selective.

Some of the infrared bands typical of the functional groups usually present in the isocyanates obtained according to this invention are indicated hereinbelow for illustrative purposes:

| | | |
|---|---|---|
| isocyanato group | 2270 | $cm^{-1}$ |
| carbodiimido group | 2120, 2140 | $cm^{-1}$ |
| urethonimino group | 1720, 1730, 1370 | $cm^{-1}$ |
| urethdione group | 1780 | $cm^{-1}$ |

Example 1
(comparative test)

A comparative test was carried out without employing catalysts, using a 2-liter glass flask equipped with a thermometer, a blade stirrer, a reflux cooler and a feeding inlet. The flask is heated to 240°C by means of an oil bath. 600 g of pure MDI (4,4'-diphenylmethane diisocyanate containing less than 3—4% of its 2,4'-isomer) in the molten state were introduced into the flask.

Charging and reaction were conducted while stirring under a dry nitrogen or carbon dioxide atmosphere.

The reaction occurred slowly. As a matter of fact, on the basis of the evolved carbon dioxide volume,

the following values of apparent NCO% and of conversion into carbodiimide at different reaction times were calculated:

| reaction time | apparent NCO% | conversion |
|---|---|---|
| 75 minutes | 32.7 | 1.85% |
| 180 " | 32 | 3.46% |
| 289 " | 31.4 | 4.9% |
| 300 " | 31.3 | 5 15% |
| 420 " | 30.5 | 6.85% |

Example 2

It was operated according to example 1, but using a 250 $cm^3$ flask and charging 240 g of pure MDI.

When the diisocyanate temperature reached, after about 15 minutes, the reaction temperature of 240°C, 30 g (12.5% referred to MDI) of commercially available molecular sieves type 3 A, in the form of small balls, were added; such catalyst had been previously subjected to a heat treatment at 300—400°C in a dry nitrogen stream.

Reaction was stopped after 6 hours by cooling.

The infrared spectroscopy of the reacted product revealed peaks relating to carbodiimides, urethonimine, urethdione and residual isocyanate.

After a 6-day aging at 40—42°C, no traces of carbodiimides were detected any longer on infrared analysis.

On the basis of the infrared bands relating to the NCO groups present in the product, there were calculated 1.85% of urethdione and 21.2% of apparent NCO, corresponding to a conversion of the starting isocyanate into carbodiimide of 25.8%.

Example 3

It was operated according to example 2, but employing 24 g (10% in respect of MDI) of commercially available molecular sieves, in the form of 3 mm (1/8" pellets), having a specific surface of 340 $m^2/g$.

Said molecular sieves had been previously activated by means of heating in a dry nitrogen stream at 300—400°C.

After a 3-hour reaction a sample was drawn and reaction was stopped by cooling.

The sample, after 13 days ageing at 25°C, appeared still liquid.

The infrared analysis revealed the presence of urethonimines, of urethdione (0.9%), of residual NCO and the absence of carbodiimides.

By titration there were determined 28.6% of apparent NCO and 30.3% of total NCO corresponding to a conversion into carbodiimide of 11%.

Another sample after a 5-hour reaction and a 25 days aging at 25°C, exhibited a I.R. titer of apparent NCO equal to 23.3% and of urethdione equal to 1.3%, corresponding to a conversion of 21.8% into carbodiimide.

Example 4

It was operated according to example 1. When after about 15—30 minutes, the temperature of the diisocyanate reached 240°C, 60 g (10% referred to MDI) of catalyst were added. The catalyst was powdered alumina (commercially available alkali-free $Al_2O_3$) having a specific surface of 140 $m^2/g$, previously activated by heating at 250—340°C in a dry nitrogen stream.

After 170 minutes, when a conversion of 29.2% was calculated on the basis of the evolved carbon dioxide, 1196 g of fresh MDI at 40—45°C were added, whereupon the entire mass was cooled down to room temperature.

The product so obtained was liquid at room temperature. Such product, separated from the catalyst simply by decantation and stored in a closed tank, remained liquid and clear for a long time without any gas evolution (meaning that carbodiimide formation was stopped).

After aging for a few days at room temperature, the infra red analysis of the product showed that functional groups such as isocyanurates, ureas and urethanes were absent. The product so obtained was therefore a MDI, the isocyanato groups thereof were partially converted into carbodiimide and successively into the urethonimino adduct, and therefore it was a modified MDI, liquid and stable at room temperature for a long time, which did not originate overpressures inside the tanks wherein it was stored, that being indicative of the fact that the carbodiimidation reaction was stopped.

The sample, after a 17-day storing, revealed a refractive index $n_D^{25}$ of 1.6179, a viscosity $\mu_{25}$ of 70 mPa.s and an infrared spectroscopic titer of apparent NCO equal to 28.9% and of urethdione equal to 0.6% corresponding to a conversion of 10% into carbodiimmide.

After a three-month storing at 20—30°C, a total NCO percentage of 30.3 was determined by titration.

After a four-month storing the product was still liquid and perfectly limpid without any traces of precipitate.

Example 5

It was operated as in example 2, using as a catalyst 24 g (10% in respect of MDI) of commercially available molecular sieves, in the form of 1.5 mm (1/16") pellets, having a specific surface of 390 $m^2/g$ and previously activated by heating to 330—450°C in a dry nitrogen stream. After a 3-hour reaction, a conversion of 12.1%, corresponding to 28.1% of apparent NCO, was calculated on the basis of the carbon dioxide evolved.

The infrared spectrum was similar to the ones of the preceding examples.

After separation of the catalyst, the product appeared liquid and limpid.

After a three-month storing at 25—30°C its appearance was unchanged.

Example 6

Example 5 was repeated, but the catalyst was activated by heating it to 160—220°C in a nitrogen stream after a pretreatment at 90—130°C with highly moist nitrogen, obtained by bubbling it through water at 90°C. Reaction was stopped by cooling after 21 minutes.

A conversion of 16.2% and an apparent NCO content of 26.3% were calculated on the basis of the volume of carbon dioxide evolved.

The infrared spectrum was similar to the ones of the preceding examples.

Example 7

It was operated as in example 4; the catalyst (60 g, 10% referred to MDI) was commercially available silica gel in granules, having a specific surface of 800 $m^2/g$ and activated by heating at 250—340°C in a dry nitrogen stream. On the basis of the gaseous carbon dioxide evolved it was possible to determine that a 10% conversion was attained after a reaction time of 269 minutes. After a 277-minute reaction time it was cooled down, thus obtaining a product which was liquid and clear at room temperature.

The catalyst was easily and rapidly removed simply by decantation.

The infrared spectrum was similar to the ones of the preceding examples.

After a 50-day storing at room temperature a refractive index $n_D^{25}$ of 1.6162 was determined.

After a 83-day storing at room temperature a viscosity $\mu_{25}$ of 54 mPa.s was determined.

Example 8

Example 7 was repeated, but the catalyst was activated by heating to 140—180°C in a dry nitrogen stream.

A 10% conversion, calculated on the basis of $CO_2$ evolved, was attained after 162 minutes.

Reaction was stopped after 165 minutes.

The infrared spectrum was quite like the ones of the preceding examples.

After a 20-day storing at room temperature, a total NCO content of 30.4% was determined.

Example 9

Example 7 was repeated, but the catalyst was activated by heating first at 120—160°C in an air stream which was previously made to bubble through water heated to 80°C, and thereafter heating the catalyst at 150—180°C in a dry air stream.

A 10% conversion, calculated on the basis of the evolved $CO_2$, was reached in 30 minutes.

The product was then diluted with 1138 g of fresh MDI heated at 40—45°C, thus obtaining, after separation of the catalyst, a liquid, limpid and fluid product.

An apparent NCO content of 28.9% was determined on the basis of the carbon dioxide evolved.

The infrared spectrum was similar to the ones of the preceding examples.

The product, stored at room temperature, exhibited after 14 days a refractive index $n_D^{25}$ of 1.6177 and after 48 days a viscosity $\mu_{25}$ of 74 mPa.s.

After two months the product was still liquid and perfectly limpid, without any precipitates.

Example 10

Example 7 was repeated, but the catalyst was activated first by heating at 100—130°C in an air stream, which was previously made to bubble through water heated at 65°C, and then heating the catalyst at 250—340°C in a dry nitrogen stream.

A 10% conversion, calculated on the basis of the evolved $CO_2$, was attained after 220 minutes.

Reaction was stopped after 333 minutes, when the conversion had reached 18.5%.

The product was then diluted with 463 g of fresh MDI heated at 40—45°C.

The final product, after removal of the catalyst, was a fluid and limpid liquid.

The infrared spectrum was similar to the ones of the preceding examples.

After a 9-day storing at room temperature the product exhibited a refractive index $n_D^{25}$ of 1.6165.

After 42 days the product was liquid and still limpid, free from any precipitate and has a viscosity $\mu_{25}$ of 56 mPa.s.

Example 11

It was operated as in example 2, but using as a catalyst, 24 g (10% in respect of MDI) of commercially available molecular sieves, in the powder form, having a specific surface of 475 $m^2/g$, and previously activated by heating it at 330—450°C in a nitrogen stream. After a 82-minute reaction, 10% of conversion was attained. Reaction was stopped after 180 minutes, when the conversion, calculated on the basis of the evolved $CO_2$, was of 14.3%. The infrared spectrum was qualitatively similar with the ones of the preceding examples.

Example 12

It was operated as in example 2, but using as a catalyst, 24 g of commercially available granular pumice stone (a complex silicate of aluminium, sodium and potassium), having a specific surface of 2 $m^2/g$ and previously heated at 250—340°C in a dry nitrogen stream.

After a 180-minute reaction, a conversion of 7.6%, determined on the basis of the $CO_2$ evolved, was attained.

The infrared spectrum of the product was qualitatively similar to the ones of the preceding examples.

Example 13
(comparative test)

It was operated as in example 2, but using as a catalyst, 24 g (10% referred to MDI) of Alundum®, a product consisting for at least 90% of $Al_2O_3$ and for the remaining part of $SiO_2$ with 0.1—0.2% of other impurities.

The catalyst was in the form of macroporous balls of 5—7 mm diameter, having a specific surface of less than 1 $m^2/g$; it had been previously activated by heating at 250—340°C in a dry nitrogen stream.

After a 76-minute reaction, a conversion of 1.3% was determined on the basis of the $CO_2$ evolved.

By comparing the results with the ones of example 1 without catalyst, where after a 75-minute reaction a conversion of 1.85% was attained, one may infer that Alundum®, compact alumina aggregated to form a macroporous mass, has no catalytic activity.

Example 14

It was operated as in example 1, but using as isocyanate, a mixture of 2,4- and 2,6- isomers (in the ratio 80/20) of toluene-diisocyanate.

60 g of catalyst (10% in respect of toluene-diisocyanate) were used, consisting of commercially available molecular sieves, in the form of 1.5 mm (1/16″) pellets, having a specific surface of 390 $m^2/g$ and previously activated by heating at 100—130°C in an air stream — which had been bubbled through water heated at 65°C — and thereafter heating the catalyst at 250—340°C in a dry nitrogen stream.

A 10% conversion, calculated on the basis of the $CO_2$ evolved, was attained after a 27-minute reaction, a 20% conversion after 56 minutes and a 30% conversion after 85 minutes.

Reaction was stopped after 150 minutes, when the conversion had reached 50%.

The product so obtained was diluted with an equal amount of fresh toluene diisocyanate. By titration a percentage of total NCO of 38.76, corresponding to a conversion of 24.7%, was determined.

The infrared analysis revealed peaks typical of an isocyanate-carbodiimide-urethonimine system.

Example 15

Example 14 was repeated, starting from pure MDI.

A 10% conversion, calculated on the basis of the $CO_2$ evolved, was attained after 28 minutes of reaction, a 20% conversion after 58 minutes and a 30% conversion after 86 minutes.

Reaction was stopped after 91 minutes when the conversion was of 31.8%.

The product was then diluted with a double amount of fresh MDI.

After removal of the catalyst, a liquid, limpid product was obtained, which had a refractive index $n_D^{25}$ of 1.6180 and a viscosity $\mu_{25}$ of 77 mPa.s.

**Claims**

1. A process for partially or thoroughly converting isocyanato groups contained in organic compounds, into carbodiimido and/or urethonimino groups by heating organic compounds containing free isocyanato functional groups at temperatures of from 140° to 300°C in the presence of catalysts, characterized in that heating is effected in the presence of a solid, inorganic catalyst of acid nature, having a specific surface of at least 2 $m^2/g$, selected from silica, alumina and mixtures and derivatives thereof wherein said two oxides are united and/or mixed with each other and/or with alkali or alkaline-earth metal oxides.

2. The process according to claim 1, in which polyisocyanates are used as organic compounds containing free isocyanato functional groups.

3. The process according to claim 2, in which 4,4′-diphenylmethane diisocyanate either alone or in admixture with its isomers and 2,4-toluene diisocyanate either alone or in admixture with its 2,6- isomer are employed as polyisocyanates.

4. The process according to any of the preceding claims, in which the catalyst consists of molecular sieves.

5. The process according to claim 1, in which heating is conducted in the presence of organic solvents not containing groups reactive towards the isocyanato groups.

**Patentansprüche:**

1. Verfahren zur teilweisen oder vollständigen Umwandlung von in organischen Verbindungen enthaltenen Isocyanatgruppen in Carbodiimid- und/oder Urethoniminogruppen durch Erhitzen organischer, freie funktionelle Isocyanatgruppen enthaltender Verbindungen auf Temperaturen von 140 bis 300°C in Anwesenheit von Katalysatoren, dadurch gekennzeichnet, daß man das Erhitzen in Anwesenheit eines festen anorganischen Katalysators saurer Natur mit einer spezifischen Oberfläche von mindestens 2 $m^2/g$, ausgewählt aus Kieselsäure, Tonerde und Mischungen und Derivaten derselben, in welchen die beiden Oxide vereinigt und/oder miteinander und/oder mit Alkali- oder Erdalkalimetalloxiden gemischt sind, durchführt.

2. Verfahren nach Anspruch 1, in welchem die Polyisocyanate als organische Verbindungen verwendet werden, die freie funktionelle Isocyanatgruppen enthalten.

3. Verfahren nach Anspruch 2, in welchem 4,4'-Diphenylmethandiisocyanat entweder allein oder in Mischung mit seinem Isomeren und 2,4-Toluoldiisocyanat entweder allein oder in Mischung mit seinem 2,6-Isomeren als Polyisocyanate verwendet werden.

4. Verfahren nach einem der vorhergehenden Ansprüche, in welchem der Katalysator aus Molekularsieben besteht.

5. Verfahren nach Anspruch 1, in welchem das Erhitzen in Anwesenheit organischer Lösungsmittel durchgeführt wird, die keine gegenüber den Isocyanatgruppen reaktionsfähigen Gruppen enthalten.

**Revendications:**

1. Procédé de conversion partielle ou totale des groupes isocyanato contenus dans des composés organiques en groupes carbodiimido et/ou uréthonimino par chauffage de composés organiques contenant des groupes fonctionnels isocyanato libres à des températures comprises entre 140 et 300°C en présence de catalyseurs, caractérisé en ce que l'on effectue le chauffage en présence d'un catalyseur minéral solide, de nature acide, dont la surface spécifique est d'au moins 2 $m^2/g$, choisi parmi la silice, l'alumine et leurs mélanges et leurs dérivés, dans lequel ces deux oxydes sont combinés et/ou mélangés l'un avec l'autre et/ou avec des oxydes de métaux alcalins ou alcalino-terreux.

2. Procédé selon la revendication 1, dans lequel on utilise les isocyanates en tant que composés organiques contenant des groupes fonctionnels isocyanato libres.

3. Procédé selon la revendication 2, dans lequel le 4,4'-diphénylméthane diisocyanate, soit seul, soit en mélange avec ses isomères et le 2,4-toluène-diisocyanate, soit seul, soit en mélange avec ses isomères 2,6-, sont employés comme polyisocyanates.

4. Procédé selon l'une quelconque des revendications précédentes, dans lequel le catalyseur est constitué de tamis moléculaires.

5. Procédé selon la revendication 1, dans lequel le chauffage est conduit en présence de solvants organiques ne contenant pas de groupes réactifs vis-à-vis des groupes isocyanato.